# EUROPEAN PATENT SPECIFICATION

(11) **EP 2 513 878 B1**
(45) Date of publication and mention of the grant of the patent: **16.10.2013**
(21) Application number: 10796137.7
(22) Date of filing: 16.11.2010
(51) Int. Cl.: G07F 11/00, A61M 16/06

(54) **SLEEP MANAGEMENT VENDING KIOSK AND ASSOCIATED METHOD**
VERKAUFSSTAND FÜR SCHLAFMANAGENEMENT UND ZUGHEÖRIGES VERFAHREN
KIOSQUE DE VENTE DE GESTION DU SOMMEIL ET PROCÉDÉ ASSOCIÉ

(30) Priority: 16.12.2009 US 286927 P
(43) Date of publication of application: 24.10.2012
(73) Proprietor: Koninklijke Philips N.V., 5656 AE Eindhoven (NL)
(72) Inventor: SMITH, David W., Briarcliff Manor New York 10510-8001 (US); SOFRANKO, Richard Andrew, Briarcliff Manor New York 10510-8001 (US); COLDREN, Kevin Anthony, Briarcliff Manor New York 10510-8001 (US)
(74) Representative: van Velzen, Maaike Mathilde
(86) International application number: PCT/IB2010/055201
(87) International publication number: WO 2011/073813

(56) References cited:
- WO-A2-2007/024694
- WO-A2-2008/036398
- US-A- 5 139 384

## Description

The present invention relates to sleep management and therapy for treating sleep disorders, such as obstructive sleep apnea (OSA), and, in particular, to a sleep management vending kiosk and associated method that provide a variety of functions relating to sleep management and therapy, including the automated provision of products for sleep management and therapy.

Across the globe there are millions of people undergoing treatment for obstructive sleep apnea (OSA). Most of those persons have been prescribed some sort of positive pressure therapy, such as continuous positive airway pressure (CPAP) therapy, for such treatment. During positive pressure therapy, a positive pressure is applied in the upper airway to splint or support the airway open, thereby preventing its collapse and the resultant airway obstruction. A typical positive airway pressure support system comprises a flow generator (e.g., a blower) that delivers gas via a delivery conduit to a respiratory patient interface device. Many respiratory patient interface devices are known in the art, and include, without limitation, nasal pillows with prongs that fit into the nares of the patient, nasal masks that fit over the patient's nose, nasal-oral masks that fit over the patient's mouth and nose, and full face masks that fit over the patient's entire face.

Within the group of patients being treated for OSA using positive pressure therapy, each patient has a unique face as well as a particular treatment modality for their level of OSA. The combination of unique facial structures and therapy pressures results in the need for a large variety of respiratory patient interface devices to accommodate the patient population. Each major manufacturer of respiratory patient interface devices has ever-increasing types & styles of product to address the uniqueness of the human face. In addition, each type/style of respiratory patient interface device typically comes in a number of sizes.

Presently, patients navigate a confusing and disjointed path to treat their OSA. Visits to primary care physicians, specialists, sleep centers, etc., are interlaced with activities to obtain new and replacement equipment though a medical device provider or other caregiver. Typically, all of the persons, facilities, and equipment needed to begin treatment for OSA are located in disparate locations, resulting in frustration to patients who want to start treatment as soon as possible.

In one exemplary embodiment, a sleep management related vending kiosk according to claim 1 is provided that includes a main housing, a processing unit provided within the main housing, a vending apparatus provided at least partially within the main housing. The vending apparatus stores a plurality of respiratory patient interface device products and is structured to selectively vend the respiratory patient interface device products under control of the processing unit. A gas flow generator is provided within the housing for delivering a flow of gas to the one of the respiratory patient interface device products to simulate actual use conditions.

In a further embodiment, a facial scanning module is provided at least partially within the main housing. The facial scanning module includes a scanning device for scanning a face of a patient. The processing unit is programmed to recommend and cause the vending apparatus to vend one of the respiratory patient interface device products based on the scanning of the face of the patient. The facial scanning module may further include a second scanning device, such as a thermal camera. The processing unit is programmed to evaluate potential for undesirable tissue conditions caused by the one of the respiratory patient interface products based on the scanning performed by the second scanning device.

In one particular embodiment, the vending kiosk also includes an electronic communications device, such as a modem, provided within the main housing and coupled to the processing unit, wherein the electronic communications device enables the sleep management related vending kiosk to electronically communicate with one or more computer systems over a network. A smart card reader may also be provided that is coupled to the processing unit and structured to read data from a smart card used by the patient in a positive pressure support system.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

FIGS. 1 and 2 are front and rear perspective views, respectively, of a sleep management vending kiosk according to one exemplary embodiment of the present invention; and

FIG. 3 is a schematic diagram of the sleep management vending kiosk of FIGS. 1 and 2 showing selected components thereof according to one particular embodiment.

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the - claims unless expressly recited therein.

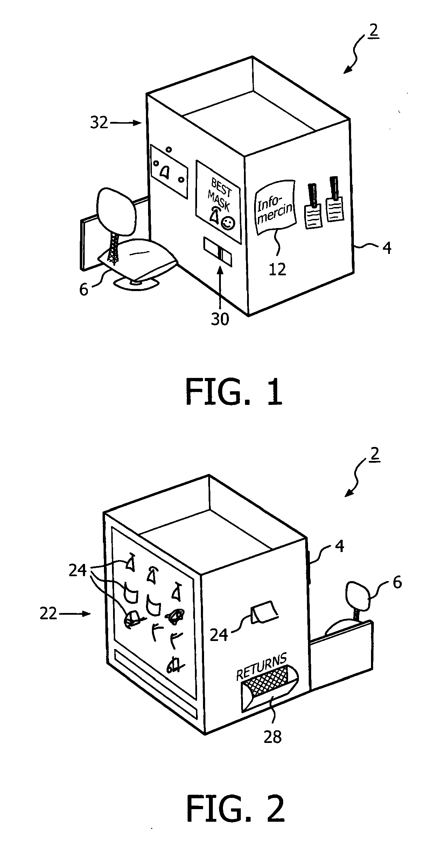

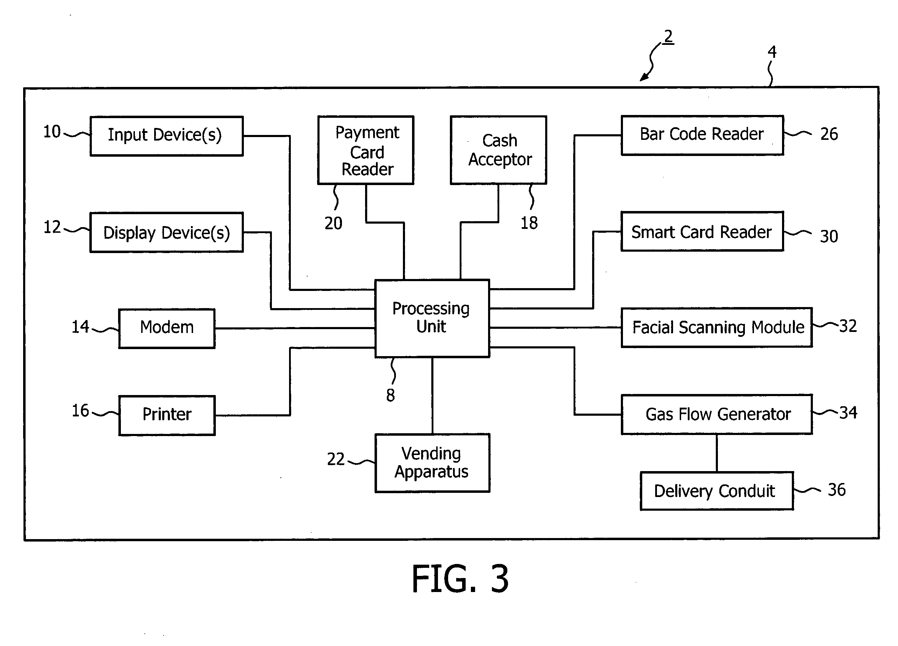

FIGS. 1 and 2 are front and rear perspective views, respectively, of a sleep management vending kiosk 2 according to one particular exemplary embodiment of the invention. FIG. 3 is a schematic (functional) diagram of sleep management vending kiosk 2 showing selected components thereof according to one particular, exemplary embodiment of the present invention. As described in greater detail herein, sleep management vending kiosk 2 is, in the exemplary embodiment, a standalone construct that can readily be accommodated within the confines of a business, such as, without limitation, a durable medical equipment (DME) provider facility, a healthcare provider (HCP) facility, or a pharmacy, or in a public venue such as a shopping mall, and that can provide a variety of functions relating to sleep management and therapy.

In the exemplary embodiment described herein, sleep management vending kiosk 2 provides functions relating to sleep management and therapy including: (1) automated control of distribution of scripted respiratory patient interface device products, (2) vending of respiratory patient interface device products, including complete devices (e.g., complete masks) and parts and accessories therefor, (3) receipt, logging and storage of returned and used products, (4) facial scanning for fitting and retrofitting of respiratory patient interface device products, (5) trouble shooting of potential tissue conditions such as red marks and discomfort that may result from therapy using particular respiratory patient interface device products, (6) uploading and downloading of smart card data (from smart cards used with positive pressure support devices such as CPAP machines and/or over wireless networks), which provides healthcare providers with the ability to track patient compliance and/or generate and provide prescriptions electrically, (7) providing information regarding sleep management and therapy such as information relating to sleep apnea and other sleep disorders, treatment product upgrades, advances in healthcare, alternative therapies, likely co-morbidities, and compliance ranking and progress, in the form of screen depictions, flyers and DVDs, and (8) streamlining the connection and communication between patients and their insurance companies or other payers.

Referring to FIGS. 1 and 2, sleep management vending kiosk 2 includes a main housing 4 that at least partially houses the components that are shown in FIG. 3. A seating area 6 is provided as part of sleep management vending kiosk 2 and is configured to accommodate one patient and physically situate the patient to have a facial scan for patient interface device fit and retrofit as described in more detail elsewhere herein. Seating area 6 can include additional seating and can include privacy curtains and other items for maintaining user privacy.

Referring to FIG. 3, sleep management vending kiosk 2 includes a processing unit 8, which may include a microprocessor, a microcontroller, or any other suitable processor, which is operatively coupled to a suitable memory for storing routines to be executed by processing unit 8. Specifically, the memory, which may be separate from and/or internal to the microprocessor, microcontroller or other suitable processor, stores one or more programs/routines for controlling the operation of sleep management vending kiosk 2 to enable it to perform the various functions and to implement the methods of operation described in greater detail elsewhere herein.

As shown in FIG. 3 and as described below, a number of additional components are included within sleep management vending kiosk 2 and operatively coupled to and under the control of processing unit 8 for providing the functionality of sleep management vending kiosk 2 described herein.

Sleep management vending kiosk 2 includes one or more input devices 10, such as a keyboard, touchscreen, voice recognition or other suitable device for enabling information to be input into sleep management vending kiosk 2 by a patient, and one or more output or display devices 12, such as an LCD display or speaker, for enabling information to be output from (i.e., displayed by) sleep management vending kiosk 2. A modem (wired or wireless) 14, or other suitable electronic (e.g., analog or digital) communications device, for enabling sleep management vending kiosk 2 to conduct data communications through a network to one or more remote computer systems. The network over which communications occur may be one or more wired and/or wireless communications networks alone or in various combinations, and may include, without limitation, the Internet.

For example, modem 14 may also be used for uploading smart card data read from a smart card used by a patient during therapy to a remote computer, such as a computer system of a healthcare provider, for analysis or for downloading data to sleep management vending kiosk 2 from a remote computer system. Such downloaded data may include electronic prescription data from a healthcare provider computer system and/or software upgrades and blower performance characteristics (for blower 34 described elsewhere herein) for processing unit 8.

In addition, modem 14 may also be used for communicating with the computer systems/databases of insurers or other payers associated with a patient in order to obtain approval (e.g., preapproval) for products offered through sleep management vending kiosk 2. For example, processing unit 8 may be programmed to navigate to the web site of an insurer/payer to confirm eligibility for products (original and replacement) that may be vended by sleep management vending kiosk 2 as described herein.

Furthermore, any payments, such as copayments under insurance plans, that may be due, may be paid at sleep management vending kiosk 2 using a cash acceptor 18 or a payment card reader 20 described below. Modem 14 may also be used for enabling sleep management vending kiosk 2 to communicate with other similar kiosks located in different, remote locations, so that patients can purchase and/or replace products as described herein as desired or needed in different global locations.

In the exemplary illustrated embodiment, sleep management vending kiosk 2 is provided with a printer 16 to enable sleep management vending kiosk 2 to print items such as coupons, customer receipts and information regarding sleep management and therapy. Cash acceptor 18 is provided for receiving cash payments, and payment card reader 20 is providing for enabling payment using payments cards, such as credit and debit cards.

A vending apparatus 22, such as a known screw vend apparatus, is also provided as part of sleep management vending kiosk 2. Vending apparatus 22 is structured to store and selectively vend, under control of processing unit 8, respiratory patient interface device products 24, including, without limitation, complete respiratory patient interface devices (e.g., complete masks), respiratory patient interface device parts and respiratory patient interface device accessories. In addition, in the exemplary embodiment, the inventory of patient interface device products 24 is automatically updated by processing unit 8 as patient interface device products 24 are vended. As described elsewhere herein, that inventory information may be provided to the various suppliers of the patient interface device products 24 so that their systems can be updated.

As seen in FIGS. 2 and 3, a bar code reader 26 is coupled to processing unit 8 and is provided to facilitate disposal and return of respiratory patient interface device products 24. In particular, return and/or disposal of respiratory patient interface device products 24 may be logged by scanning a bar code provided thereon or therewith (e.g., on packaging) to identify the particular item and then the particular item may be deposited in a receiving bin 28, which shuttles it to a holding area for subsequent shipping, storage or destruction. As respiratory patient interface device products 24 are returned in this manner, the inventory of patient interface device products 24 is automatically updated by processing unit 8. The receiving bin 28 also provides a mechanism to leverage eco-friendly disposal of used patient interface device products 24.

Sleep management vending kiosk 2 further includes a smart card reader 30. As discussed briefly above, smart card reader 30 is structured to read data from and write data to smart cards, such as smart cards that are commonly used with positive pressure support systems such as CPAP machines. As a result, a patient that uses a positive pressure support system, such as a CPAP machine, can bring the smart card that they have been using to log usage and therapy data on that device to sleep management vending kiosk 2, wherein the stored data can be read from the smart card using smart card reader 30 and immediately or subsequently transmitted (uploaded) to a healthcare provider (i.e., their computer system) so that the healthcare provider can track patient compliance.

In addition, in the exemplary embodiment, the healthcare provider is provided with a compliance management system, such as the ENCORE® Compliance Management System offered by the Philips, for allowing the healthcare provider to assess patient compliance, review therapy data and maintain patient therapeutic histories. Reports from the compliance management system that provide graphical and statistical analysis of the data may then be downloaded to sleep management vending kiosk 2 and printed for the patient using printer 16. In an alternative embodiment, the sleep management vending kiosk 2 (and in particular the processing unit 8) is provided with a compliance management system, , such as the Encore system, for locally analyzing data read from smart cards by smart card reader device 30.

Sleep management vending kiosk 2 also includes a facial scanning module 32 that is, in the exemplary embodiment, structured to, in conjunction with software provided in processing unit 8, provide a mechanism for fitting or refitting a patient with a respiratory patient interface device (e.g., one of the respiratory patient interface devices 24) and/or for trouble shooting of tissue conditions such as red marks or discomfort resulting from use of a respiratory patient interface device (e.g., one of the respiratory patient interface devices 24). More particularly, in the exemplary embodiment, facial scanning module 32 includes a scanning device, such as, without limitation, a charge coupled device (CCD), a CMOS active-pixel sensor, or another digital image sensor, for capturing an image of the patient's face and the software provided in processing unit 8 is adapted to automatically determine a particular respiratory patient interface device that would be a good fit for the patient based on the captured image.

For example, the software may be provided with information, such as a table, that correlates certain facial measurements with certain recommended respiratory patient interface devices 24, and the software may be further adapted to measure certain features of the patient's face using the captured image (e.g., like know facial recognition software) and then recommended one or more respiratory patient interface devices 24 based on the stored correlations. U.S. patent application no. 11/150,860 (publication no. 2006/0023228), discloses an imaging system for creating a custom patient interface device, such as a mask, suitable for use with the present invention.

The present invention further contemplates that the facial scanning module can be used to capture and/or generate a three-dimensional image or three-dimensional anatomic data regarding the user's face or facial features. This is accomplished using digital cameras and/or laser scanning systems as known to those skilled in the art.

In addition, in the exemplary embodiment, facial scanning module 32 further includes a thermal camera for capturing thermal images of the patient's face and the software provided in processing unit 8 is adapted to automatically evaluate the likelihood of whether a particular respiratory patient interface device will lead to undesirable tissue conditions, such as pressure sores or discomfort, during use based on captured thermal images of the patient's face before an after wearing a particular device. In one particular embodiment, the thermal camera and associated software are implemented in accordance with the methodologies described in United States Provisional Application No 61/260,039, owned by Philips, entitled "Method of Evaluating Potential for a Pressure Sore or Discomfort of a Patient Caused by an Object on the Patient".

Sleep management vending kiosk 2 also includes a gas flow generator 34, such as a blower used in a conventional CPAP or bi-level pressure support device, which is coupled to delivery conduit 36. Gas flow generator 34 receives a breathing gas from any suitable source, such as the ambient atmosphere, and generates a flow of breathing gas generally equal to or above ambient atmospheric pressure that may be delivered to a respiratory patient interface device product 24 that is coupled to delivery conduit 36. Thus, by including gas flow generator 34 and delivery conduit 36 in the exemplary embodiment of sleep management vending kiosk 2, a patient may be fit for, trouble shoot and test various respiratory patient interface devices, e.g., respiratory patient interface device products 24 vended from vending apparatus 22, under actual use conditions with breathing gasses being supplied thereto.

Having described the various components and features of sleep management vending kiosk 2, a process by which a patient may obtain a respiratory patient interface device product 24 from sleep management vending kiosk 2 according to the exemplary embodiment will now be described. In operation, a patient can initiate such a process by entering personal information into sleep management vending kiosk 2 using one of the input devices 10 such as keyboard. Such personal information may include insurer or other payer information to enable sleep management vending kiosk 2 to obtain the necessary approvals as described elsewhere herein as needed. In addition, any required payments (e.g., full payment for a respiratory patient interface device product 24 to be vended or a copayment therefore that is associated with an insurer or other payer) may be made using cash acceptor 18 or payment card reader 20 at that time (if the fees are able to be determined) or later in the process if necessary.

The patient may then be instructed on one of the display devices 12 to be seated in the seating area 6 so that an initial scan of the patient's face may be obtained using a facial scanning module 32. Based on the initial scan (using he image scanner described above), an initial recommendation for a particular respiratory patient interface device product 24 will be made by sleep management vending kiosk 2, and that recommended respiratory patient interface device product 24 will be vended from vending apparatus 22 under control of processing unit 8. In the exemplary embodiment, the inventory of respiratory patient interface device products 24 maintained by sleep management vending kiosk 2 will be updated at the time of vending and adjusted as needed on the basis of any returns as described below. Such inventory information may be reported directly to the associated suppler by sleep management vending kiosk 2 through modem 14, or, alternatively may be collected and aggregated (e.g., with information from other similar kiosks) by the provider of sleep management vending kiosk 2 and later reported to the associated supplier or suppliers.

The patient may then put the recommended respiratory patient interface device product 24 on his or her face and try it out for overall fit and comfort, utilizing on screen instructions provided by sleep management vending kiosk 2 on one of the display devices 12. As noted elsewhere herein, in the exemplary embodiment, the patient may try the recommended respiratory patient interface device product 24 while it is connected to delivery conduit 36 so that gas may be delivered thereto by gas flow generator 34 to simulate actual use conditions. In addition, the recommended respiratory patient interface device product 24 may also be evaluated for potential adverse tissue conditions and discomfort problems using the thermal scanning aspect of facial scanning module 32 as described elsewhere herein. The results of that evaluation may be provided on one of the display devices 12.

If the initially recommended respiratory patient interface device product 24 is unsatisfactory, based on either the patient's subjective evaluation of fit and comfort and/or on the results of the thermal scanning, the patient may return the respiratory patient interface device product 24 to the sleep management vending kiosk 2 by scanning a bar code provide therewith using bar code scanner 26 and inserting it into receiving bin 28. The scanning of the bar code at this stage allows sleep management vending kiosk 2 to properly track inventory and mark the recommended respiratory patient interface device product 24 as a returned item. The patient may then repeat the steps described above until a satisfactory respiratory patient interface device product 24 is obtained. The patient may also, at that time or at a later time, purchase any desired parts or accessories from vending apparatus 22, with payment being made using cash acceptor 18 or payment card reader 20.

Thus, as described in detail above, sleep management vending kiosk 2 in the exemplary embodiment provides a single, centralized, standalone mechanism that provides a variety of functions relating to sleep management and therapy including, without limitation, automated storage, distribution and inventory control and vending of respiratory patient interface device products (including complete devices, parts and accessories), fitting and trouble shooting of such products, reading and uploading and downloading of smart card data, the provision of sleep management and therapy information, and streamlined connectivity and communication between patients and their insurance companies.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A sleep management related vending kiosk (2), comprising:
a main housing (4);
a processing unit (8) provided within the main housing; and
a vending apparatus (22) provided at least partially within the main housing, the vending apparatus storing a plurality of respiratory patient interface device products (24) and being structured to selectively vend the respiratory patient interface device products under control of the processing unit, **characterized in that**
the sleep management related vending kiosk further comprises a gas flow generator (34) provided within the housing and a delivery conduit (36) coupled to the gas flow generator, wherein the delivery conduit is structured to be coupled to and deliver a flow of gas from the gas flow generator to one of the respiratory patient interface device products.

2. The sleep management related vending kiosk according to claim 1, further comprising a facial scanning module (32) provided at least partially within the main housing, the facial scanning module including a scanning device for scanning a face of a patient, wherein the processing unit is programmed to recommend and cause the vending apparatus to vend one of the respiratory patient interface device products based on the scanning of the face of the patient.

3. The sleep management related vending kiosk according to claim 2, wherein the scanning device comprises a digital image scanner structured to generate a digital image of the face of the patient, and wherein the processing unit is programmed to recommend the one of the respiratory patient interface device products based on the digital image.

4. The sleep management related vending kiosk according to claim 2, wherein the facial scanning module further includes a second scanning device, and wherein the processing unit is programmed to evaluate potential for undesirable tissue conditions caused by the one of the respiratory patient interface products based on the scanning performed by the second scanning device.

5. The sleep management related vending kiosk according to claim 4, wherein the second scanning device is a thermal camera for capturing a plurality of thermal images of the face of the patient, and wherein the processing unit is programmed to evaluate potential for undesirable tissue conditions caused by the one of the respiratory patient interface based on the thermal images.

6. The sleep management related vending kiosk according to claim 1, further comprising a bar code reader (26) coupled to the processing unit and structured to read bar codes associated with the respiratory patient interface device products, and wherein the main housing further includes a receiving bin (28) for receiving returned ones of the respiratory patient interface device products after having been vended by the vending apparatus.

7. The sleep management related vending kiosk according to claim 1, further comprising a smart card reader (30) coupled to the processing unit and structured to read data from a smart card used by the patient in a positive pressure support system.

8. The sleep management related vending kiosk according to claim 7, wherein the processing unit is provided with a compliance management system for analyzing data read from the smart card by the smart card reader.

9. The sleep management related vending kiosk according to claim 1, further comprising an electronic communications device (14) provided within the main housing and coupled to the processing unit, the electronic communications device enabling the sleep management related vending kiosk to electronically communicate with a computer system over a network.

10. The sleep management related vending kiosk according to claim 9, further comprising a smart card reader (30) coupled to the processing unit and structured to read data from a smart card used by the patient in a positive pressure support system, wherein the processing unit is programmed to transmit the data read from the smart card to the computer system over the network.

11. The sleep management related vending kiosk according to claim 9, wherein the computer system includes a compliance management system for analyzing the data read from the smart card and generating compliance data based thereon, and wherein the processing unit is programmed to receive the compliance data from the computer system over the network and cause a report based on the compliance data to be provided to the patient.

12. The sleep management related vending kiosk according to claim 8, wherein the computer system is associated with:
(a) an insurer or payer associated with the patient, and wherein the processing unit is programmed to communicate with the computer system to obtain approval for a selected one of the respiratory patient interface device products;
(b) a supplier of one or more of the respiratory patient interface device products, and wherein the processing unit is programmed to communicate inventory information relating to one or more of the respiratory patient interface device products to the computer system; or
(c) a healthcare provider, and wherein the processing unit is programmed to receive digital prescription information from the computer system over the network.

13. The sleep management related vending kiosk according to claim 1, further comprising either or both of a payment card reader provided at least partially within the main housing and coupled to the processing unit and a cash acceptor provided at least partially within the main housing and coupled to the processing unit.

14. A method of assisting a patient with sleep management therapy, comprising:
providing a vending kiosk (2);
storing a plurality of respiratory patient interface device products (24) in the vending kiosk;
selecting a respiratory patient interface device product via an input device (10) associated with the vending kiosk;
dispensing the selected respiratory patient interface device product from the vending kiosk to the patient; and charecterized by
generating a flow of gas in the vending kiosk and delivering the flow of gas from the vending kiosk to the recommended one of the respiratory patient interface device products.

15. The method according to claim 14, further comprising:
scanning the patient's face using the vending kiosk;
determining, in the vending kiosk, a recommended one of the respiratory patient interface device products based on the scanning of the face of the patient; and
providing the recommended one of the respiratory patient interface device products to the patient from the vending kiosk.

## Patentansprüche

1. Verkaufsautomat (2) für Schlafmanagement, der Folgendes umfasst:
eine Haupteinhausung (4),
einen Prozessor (8), der sich in der Haupteinhausung befindet, und
eine Verkaufsvorrichtung (22), die zumindest teilweise in der Haupteinhausung untergebracht ist, wobei die Verkaufsvorrichtung eine Vielzahl von Atemwegsschnittstellenprodukten (24) für Patienten lagert und so ausgelegt ist, dass sie gesteuert von dem Prozessor selektiv die Atemwegsschnittstellenprodukte verkauft, **dadurch gekennzeichnet, dass**
der Verkaufsautomat für Schlafmanagement ferner Folgendes umfasst:
einen Gasflussgenerator (34), der in der Einhausung untergebracht ist, und eine mit dem Gasflussgenerator verbundene Zuführleitung (36), wobei die Zuführleitung so ausgelegt ist, dass sie mit dem Gasflussgenerator verbunden ist und von dem Gasflussgenerator zu einem der Atemwegsschnittstellenprodukte für Patienten einen Gasfluss zuführt.

2. Verkaufsautomat für Schlafmanagement nach Anspruch 1, der ferner ein Gesichtsscanmodul (32) umfasst, das zumindest teilweise in der Haupteinhausung untergebracht ist, wobei das Gesichtsscanmodul einen Scanner zum Scannen des Gesichts eines Patienten umfasst, wobei der Prozessor so programmiert ist, dass er dem Verkaufsautomaten empfiehlt und ihn veranlasst, auf der Grundlage des Gesichtsscans des Patienten eines der Atemwegsschnittstellenprodukte für Patienten zu verkaufen.

3. Verkaufsautomat für Schlafmanagement nach Anspruch 2, wobei der Scanner einen digitalen Bildscanner umfasst, der so ausgelegt ist, dass er ein digitales Bild des Gesichts des Patienten erzeugt, und wobei der Prozessor so programmiert ist, dass er auf der Grundlage des digitalen Bildes das eine der Atemwegsschnittstellenprodukte für Patienten empfiehlt.

4. Verkaufsautomat für Schlafmanagement nach Anspruch 2, wobei das Gesichtsscanmodul ferner einen zweiter Scanner umfasst und wobei der Prozessor so programmiert ist, dass er auf der Grundlage des durch den zweiten Scanner durchgeführten Scan die Möglichkeit von durch das eine der Atemwegsschnittstellenprodukte für Patienten verursachten unerwünschten Gewebebedingungen auswertet.

5. Verkaufsautomat für Schlafmanagement nach Anspruch 4, wobei der zweite Scanner eine Wärmekamera zur Erfassung einer Vielzahl von Wärmebildern des Gesichts des Patienten ist, und wobei der Prozessor so programmiert ist, dass er auf der Grundlage der Wärmebilder die Möglichkeit von durch das eine der Atemwegsschnittstellenprodukte für Patienten verursachten unerwünschten Gewebebedingungen auswertet.

6. Verkaufsautomat für Schlafmanagement nach Anspruch 1, der ferner einen Barcodeleser (26) umfasst, der mit dem Prozessor verbunden und so ausgelegt ist, dass er Barcodes liest, die zu Atemwegsschnittstellenprodukten für Patienten gehören, und wobei die Haupteinhausung ferner einen Aufnahmebehälter (28) zur Aufnahme zurückgegebener Atemwegsschnittstellenprodukte umfasst, nachdem sie von dem Verkaufsautomaten verkauft wurden.

7. Verkaufsautomat für Schlafmanagement nach Anspruch 1, der ferner einen Chipkartenleser (30) umfasst, der mit dem Prozessor verbunden und so ausgelegt ist, dass er Daten von einer Chipkarte liest, die von dem Patienten in einem Überdruckunterstützungssystem verwendet wird.

8. Verkaufsautomat für Schlafmanagement nach Anspruch 7, wobei der Prozessor mit einem Compliance-Management-System zur Analyse der durch den Chipkartenleser aus der Chipkarte gelesenen Daten ausgestattet ist.

9. Verkaufsautomat für Schlafmanagement nach Anspruch 1, der ferner eine elektronische Kommunikationsvorrichtung (14) umfasst, die sich in der Haupteinhausung befindet und mit dem Prozessor verbunden ist, wobei die elektronische Kommunikationsvorrichtung den Verkaufsautomaten für Schlafmanagement in die Lage versetzt, über ein Netzwerk elektronisch mit einem Computersystem zu kommunizieren.

10. Verkaufsautomat für Schlafmanagement nach Anspruch 9, der ferner einen Chipkartenleser (30) umfasst, der mit dem Prozessor verbunden und so ausgelegt ist, dass er Daten von einer Chipkarte liest, die von dem Patienten in einem Überdruckunterstützungssystem verwendet wird, wobei der Prozessor so programmiert ist, dass er die aus der Chipkarte gelesenen Daten über das Netzwerk an das Computersystem sendet.

11. Verkaufsautomat für Schlafmanagement nach Anspruch 9, wobei das Computersystem ein Compliance-Management-System zur Analyse der aus der Chipkarte gelesenen Daten und auf deren Grundlage zur Erzeugung von Compliance-Daten umfasst, und wobei der Prozessor so programmiert ist, dass er die Compliance-Daten über das Netzwerk von dem Computersystem empfängt und veranlasst, dass der Patient einen Bericht auf der Grundlage der Compliance-Daten erhält.

12. Verkaufsautomat für Schlafmanagement nach Anspruch 8, wobei das Computersystem einer der folgenden Institutionen zugehörig ist:
(a) einem Versicherer oder Kostenträger, der dem Patienten zugeordnet ist, wobei der Prozessor so programmiert ist, dass er mit dem Computersystem kommuniziert, um die Genehmigung für das ausgewählte Atemwegsschnittstellenprodukt zu erhalten,
(b) einem Lieferanten von einem oder mehreren der Atemwegsschnittstellenprodukte, wobei der Prozessor so programmiert ist, dass er dem Computersystem Bestandsinformationen zu dem einen oder mehreren Atemwegsschnittstellenprodukten übermittelt, oder
(c) einem Gesundheitsdienstleister, wobei der Prozessor so programmiert ist, dass er über das Netzwerk von dem Computersystem digitale Verschreibungsinformationen erhält.

13. Verkaufsautomat für Schlafmanagement nach Anspruch 1, der ferner entweder einen Zahlungskartenleser, der sich zumindest teilweise in der Haupteinhausung befindet und mit dem Prozessor verbunden ist, oder eine Bargeldannahmevorrichtung, die sich zumindest teilweise in der Haupteinhausung befindet und mit dem Prozessor verbunden ist, oder beides umfasst.

14. Verfahren zur Unterstützung eines Patienten bei einer Therapie zum Schlafmanagement, das Folgendes umfasst:
Bereitstellen eines Verkaufsautomaten (2),
Lagern einer Vielzahl von Atemwegsschnittstellenprodukten (24) für Patienten in dem Verkaufsautomaten,
Auswählen eines Atemwegsschnittstellenprodukts für Patienten über eine Eingabevorrichtung (10), die zu dem Verkaufsautomaten gehört,
Ausgeben des ausgewählten Atemwegsschnittstellenprodukts für Patienten aus dem Verkaufsautomaten an den Patienten, **gekennzeichnet durch**
Erzeugen eines Gasflusses in dem Verkaufsautomaten und Zuführen des Gasflusses von dem Verkaufsautomaten zu dem empfohlenen der Atemwegsschnittstellenprodukte für Patienten.

15. Verfahren nach Anspruch 14, das ferner Folgendes umfasst:
Scannen des Gesichts des Patienten mit Hilfe des Verkaufsautomaten,
Ermitteln in dem Verkaufsautomaten eines empfohlenen Atemwegsschnittstellenprodukts für Patienten auf der Grundlage des Gesichtsscans des Patienten und
Bereitstellen des empfohlenen Atemwegsschnittstellenprodukts für Patienten von dem Verkaufsautomaten für den Patienten.

## Revendications

1. Kiosque de vente associé à la gestion du sommeil (2), comprenant :
un logement principal (4) ;
une unité de traitement (8) ménagée dans le logement principal ; et
un appareil de vente (22) fourni au moins partiellement dans le logement principal, l'appareil de vente stockant une pluralité de produits de dispositifs d'interface respiratoire de patient (24) et étant structuré de façon à vendre sélectivement les produits des dispositifs d'interface respiratoire de patient sous le contrôle de l'unité de traitement, **caractérisé en ce que**
le kiosque de vente associé à la gestion du sommeil comprend en outre un générateur de flux gazeux (34) fourni dans le logement et un conduit de distribution (36) raccordé au générateur de flux gazeux, dans lequel le conduit de distribution est structuré de façon à être raccordé et à fournir un flux gazeux provenant du générateur de flux gazeux à un des produits des dispositifs d'interface respiratoire du patient.

2. Kiosque de vente associé à la gestion du sommeil selon la revendication 1, comprenant en outre un module de balayage facial (32) ménagé au moins partiellement dans le logement principal, le module de balayage facial comprenant un dispositif de balayage pour scanner le visage d'un patient, dans lequel l'unité de traitement est programmée pour recommander et faire en sorte que l'appareil de vente vende un des produits des dispositifs d'interface respiratoire du patient, sur la base du balayage du visage du patient.

3. Kiosque de vente associé à la gestion du sommeil selon la revendication 2, dans lequel le dispositif de balayage comprend un scanner d'image numérique structuré de façon à générer une image numérique du visage du patient, et dans lequel l'unité de traitement est programmée pour recommander l'un des produits des dispositifs d'interface respiratoire du patient, sur la base de l'image numérique.

4. Kiosque de vente associé à la gestion du sommeil selon la revendication 2, dans lequel le module de balayage facial comprend en outre un second dispositif de balayage, et dans lequel l'unité de traitement est programmée pour évaluer le potentiel pour un état de tissu indésirable provoqué par l'un des produits d'interface respiratoire du patient sur la base du balayage réalisé par le second dispositif de balayage.

5. Kiosque de vente associé à la gestion du sommeil selon la revendication 4, dans lequel le second dispositif de balayage est une caméra thermique pour capturer une pluralité d'images thermiques du visage du patient, et dans lequel l'unité de traitement est programmée pour évaluer le potentiel un état de tissu indésirable provoqué par l'interface respiratoire du patient, sur la base des images thermiques.

6. Kiosque de vente associé à la gestion du sommeil selon la revendication 1, comprenant en outre un lecteur de code-barres (26) raccordé à l'unité de traitement et structuré pour lire les codes-barres associés aux produits du dispositif d'interface respiratoire du patient, et dans lequel le logement principal comprend en outre un bac de réception (28) pour recevoir les produits de dispositif d'interface respiratoire du patient renvoyés après qu'ils aient été vendus par l'appareil de vente.

7. Kiosque de vente associé à la gestion du sommeil selon la revendication 1, comprenant en outre un lecteur de carte à puces (30) raccordé à l'unité de traitement et structuré pour lire les données à partir d'une carte à puces utilisée par le patient dans un système de support à pression positive.

8. Kiosque de vente associé à la gestion du sommeil selon la revendication 7, dans lequel l'unité de traitement est dotée d'un système de gestion de la conformité pour analyser les données lues sur la carte à puces par le lecteur de carte à puces.

9. Kiosque de vente associé à la gestion du sommeil selon la revendication 1, comprenant en outre un dispositif de communication électronique (14) ménagé dans le logement principal et raccordé à l'unité de traitement, le dispositif de communication électronique permettant au kiosque de vente associé à la gestion du sommeil de communiquer électroniquement avec un système informatique sur un réseau.

10. Kiosque de vente associé à la gestion du sommeil selon la revendication 9, comprenant en outre un lecteur de carte à puces (30) raccordé à l'unité de traitement et structuré pour lire des données d'une carte à puces utilisée par le patient dans un système de support à pression positive, dans lequel l'unité de traitement est programmée pour transmettre les données lues de la carte à puce au système informatique sur le réseau.

11. Kiosque de vente associé à la gestion du sommeil selon la revendication 9, dans lequel le système informatique comprend un système de gestion de la conformité pour analyser les données lues sur la carte à puce et générer les données de conformité basées dessus, et dans lequel l'unité de traitement est programmée pour recevoir les données de conformité provenant du système informatique sur le réseau et provoquer un rapport sur la base des données de conformité à fournir au patient.

12. Kiosque de vente associé à la gestion du sommeil selon la revendication 8, dans lequel le système informatique est associé à :
(a) un assureur ou payeur associé au patient, et dans lequel l'unité de traitement est programmée pour communiquer avec le système informatique afin d'obtenir l'approbation pour un élément sélectionné parmi des produits de dispositif d'interface respiratoire du patient ;
(b) un fournisseur d'un ou plusieurs des produits de dispositif d'interface respiratoire du patient et dans lequel l'unité de traitement est programmée pour communiquer des informations de stock concernant un ou plusieurs des produits de dispositif d'interface respiratoire du patient au système informatique ; ou
(c) un prestataire de soins et dans lequel l'unité de traitement est programmée pour recevoir des informations d'une prescription numérique provenant du système informatique sur le réseau.

13. Kiosque de vente associé à la gestion du sommeil, selon la revendication 1, comprenant en outre un lecteur de carte de paiement, ménagé au moins partiellement dans le logement principal et raccordé à l'unité de traitement ou un accepteur de monnaie fourni au moins partiellement dans le logement principal et raccordé à l'unité de traitement, ou les deux.

14. Procédé d'assistance à un patient avec un traitement de gestion du sommeil, comprenant :
la fourniture d'un kiosque de vente (2) ;
le stockage d'une pluralité de produits de dispositif d'interface respiratoire du patient (24) dans le kiosque de vente ;
la sélection d'un produit de dispositif d'interface respiratoire du patient via un dispositif d'entrée (10) associé au kiosque de vente ;
la distribution du produit de dispositif d'interface respiratoire du patient sélectionné du kiosque de vente au patient et **caractérisé par**
la génération d'un flux gazeux dans le kiosque de vente et la fourniture du flux gazeux depuis le kiosque de vente à l'un des produits du dispositif d'interface respiratoire du patient.

15. Procédé selon la revendication 14, comprenant en outre :
le balayage du visage du patient en utilisant le kiosque de vente ;
la détermination, dans le kiosque de vente, d'un élément recommandé parmi les produits de dispositif d'interface respiratoire du patient basé sur le balayage du visage du patient ; et
la fourniture de l'un des produits recommandés du dispositif d'interface respiratoire du patient audit patient depuis le kiosque de vente.
